(19) **Europäisches Patentamt / European Patent Office / Office européen des brevets**

(11) **EP 3 085 790 B1**

(12) # EUROPEAN PATENT SPECIFICATION

(45) Date of publication and mention
of the grant of the patent:
**29.08.2018 Bulletin 2018/35**

(51) Int Cl.:
*C12Q 1/02* (2006.01)     *C12Q 1/18* (2006.01)
*C12R 1/445* (2006.01)

(21) Application number: **14872796.9**

(22) Date of filing: **11.12.2014**

(86) International application number:
**PCT/CL2014/000072**

(87) International publication number:
**WO 2015/089683 (25.06.2015 Gazette 2015/25)**

(54) **METHOD FOR THE MICROBIOLOGICAL DETERMINATION OF TRACES OF ANTIBIOTICS IN LOW VOLUME BIOLOGICAL SAMPLES**

VERFAHREN ZUR MIKROBIOLOGISCHEN BESTIMMUNG VON SPUREN VON ANTIBIOTIKA IN BIOLOGISCHEN PROBEN MIT GERINGEM VOLUMEN

PROCÉDÉ DE DÉTERMINATION MICROBIOLOGIQUE DE TRACES D'ANTIBIOTIQUES DANS DES ÉCHANTILLONS BIOLOGIQUES DE FAIBLE VOLUME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.12.2013 CL 2013003628**

(43) Date of publication of application:
**26.10.2016 Bulletin 2016/43**

(73) Proprietor: **Universidad De Santiago De Chile Estacion Central,
Santiago (CL)**

(72) Inventors:
• **SANHUEZA CHAVEZ, Loreto**
**Santiago (CL)**
• **WILKENS ANWANDTER, Marcela Andrea**
**Santiago (CL)**
• **LAURIDO FUENZALIDA, Claudio Aurelio**
**Santiago (CL)**

(74) Representative: **Isern Patentes y Marcas S.L.
Paseo de la Castellana 115, 1° dcha
28046 Madrid (ES)**

(56) References cited:
**EP-A2- 0 418 113     WO-A2-2005/108626**

• **Eucast ET AL: "Determination of minimum inhibitory concentrations (MICs) of antibacterial agents by broth dilution", Clinical Microbiology and Infection, 1 August 2003 (2003-08-01), pages 1-7, XP055369300, Retrieved from the Internet: URL:http://onlinelibrary.wiley.com/store/1 0.1046/j.1469-0691.2003.00790.x/asset/j.14 69-0691.2003.00790.x.pdf?v=1&t=j2a4xfoi&s= 9a7ad466b2b8af7e730cc128b6b597ba8d30d1e9 [retrieved on 2017-05-04]**
• **T.O. OMOTADE ET AL: "D-cycloserine or similar physiochemical compounds may be uniquely suited for use in Bacillus anthracis spore decontamination strategies", JOURNAL OF APPLIED MICROBIOLOGY., vol. 115, no. 6, 30 August 2013 (2013-08-30), pages 1343-1356, XP055369346, GB ISSN: 1364-5072, DOI: 10.1111/jam.12322**
• **EDBERG S. C. ET AL.: 'Turbidimetric determination of blood amino glycoside levels by growth curve analysis.' JOURNAL OF PHARMACEUTICAL SCIENCES vol. 69, no. 12, 1980, pages 1442 - 1443, XP055353000**
• **WHYATT P. L. ET AL.: 'Rapid, Precise, Turbidometric Assay for Low Levels of Ampicillin in Serum After Single-Dose Oral Administration.' ANTIMICROB AGENTS CHEMOTHER. vol. 6, no. 6, 1974, pages 811 - 814, XP055353001**

**(Cont. next page)**

- EDBERG, S. C.: 'Determination of antibiotics levels in human body fluids: techniques and significance bactericidal tests in endo carditis and other severe infections.' ANTIBIOTICS IN LABORATORY MEDICINE. vol. 206-264, 1980, USA, pages 225 - 228
- LOURENÇO F. R. ET AL.: 'Antibiotic microbial assay using kinetic-reading microplate system.' BRAZILIAN JOURNAL OF PHARMACEUTICAL SCIENCES vol. 47, no. 3, 2011, pages 573 - 584, XP055353004
- ØSTERGAARD C. ET AL.: 'Evaluation of Moxifloxacin, a New 8-Methoxyquinolone, for Treatment of Meningitis Caused by a Penicillin Resistant Pneumococcus in Rabbits.' ANTIMICROBIAL AGENTS AND CHEMOTHERAPY vol. 42, no. 7, 1998, pages 1706 - 1712, XP055353005

## Description

### Field of the invention

The present invention relates to a microbiological method for determining traces of antibiotics in low volume biological samples.

### Background of the invention

Numerous studies have confirmed the role of spinal glia in the development and persistence of chronic pain. Consistent with this, it is possible to propose that pharmacological suppression of glial function in the spinal cord may depress spinal mechanisms of pain. Different drugs have shown that, besides acting as antibiotics (minocycline, amoxicillin, neomycin, ceftriaxone), they exhibit antiinflammatory activity. This makes drugs useful in studies of chronic pain. Usually, these antibiotics are administered via intrathecal to rats having chronic pain conditions, to a compartment having a very low volume (about 80-100 $\mu$L) in these animals. Determining these antibiotics by classical methods such as high performance liquid chromatography (HPLC) is a great challenge, as in this case the sample should be cleared of elements present in the cerebrospinal fluid, such as proteins, peptides or other components preventing their use in HPLC chromatography. Moreover, kinetic studies of antibiotics could not be accomplished, because only one sample for HPLC consumes all the cerebrospinal fluid of the rat.

In addition, this methodology can be used for determining antibiotics in other biological fluids such as urine, blood, synovial fluid, eye aqueous humor, etc.

It is also likely that, using HPLC coupled to mass spectrometry, traces of antibiotics in biological samples or others could be detected, but the volume required for detection is high (in the range of mL). Microbiological method is a simple, sensitive and cost-saving alternative compared with determinations made by the HPLC method.

Specifically, in prior art, an assay to determine blood levels of antibiotic (kanamycin and gentamycin) measured in 40 minutes to 1.5 hours using samples of 0.1-0.6 ml of serum, has been disclosed. The principle is based on the measurement of pH drop in a culture containing a heavy inoculum of test organisms. Bacterial growth is inhibited by antibiotics, resulting in the inhibition of fermentation. The degree of inhibition is reflected in the pH change in the culture medium, which is related to the concentration of added antibiotics. See "Rapid microbiological assay of antibiotics in blood and other body fluids" by S. Faine and D.C. Knight in The Lancet, August 17, 1968, pages 375-378

An assay for screening levels of antibiotics in cow's milk, by using Delvotest SP-NT and Copan Milk Test is also known. These inhibitory assays consist of agar ampoules or microplates containing spores of *Bacillus stearothermophilus* and nutrients. With the addition of the milk under study, the spores germinate and produce carbonic acid. This acid causes the bromocresol purple indicator in the ampoule to change color from purple to yellow. Thus, a yellow ampoule after incubation indicates a negative sample. The presence of antibiotics inhibits bacterial growth and the color of the agar in the ampoule remains purple, indicating a positive result. The Delvotest SP-NT and the Copan Milk Test were developed from SP Delvotest and Delvotest Milk Control Stations (MCS). See Validation and comparison of the Copan Milk Test and Delvotest SP-NT for the detection of antimicrobials in milk. Marie-Hélène Le Breton, Marie-Claude Savoy-Perroud Jean-Marc Diserens. Analytica Chimica Acta 586 (1-2): 280-283, (2007).

Another known assay is based on the concentration-dependent variation of the inhibitory effect of antibiotics on reference bacteria, *B. subtilis* ATCC 6633, *S. aureus* ATCC 6538p and *S. epidermidis* ATCC 12228, in a seeded agar. Results were expressed as average inhibition zone (in mm) *versus* antibiotic concentration. See Application of microbiological assay to determine pharmaceutical equivalence of generic intravenous antibiotics. Andres F. Zuluaga, Maria Agudelo, Carlos Rodriguez and Omar Vesga. BMC Clinical Pharmacology 2009, 9: 1 doi: 10, 1186/1472-6904-9-1.

Moreover, CN1858232 discloses a technology for detecting antibiotic residues in agricultural products, particularly a method of detecting microbiological sulfa drugs residues in edible animal tissue developed in kit form. The method comprises preparing a culture medium I and a culture medium II; forming a culture medium with culture medium I and culture medium II, it uses *Bacillus megaterium* as work microorganism, neomycin paper sheets for quality control and synergistic trimethoprim solution. The technology provides simple detection, high sensitivity, and long shelf life of the kit.

EP0418113 relates to a microbiological test kit and method for detecting antibacterial compounds in a specimen, particularly useful for food or dairy industry. Said method comprising the step of: a) admixing said species with a viable bacterium exhibiting enhanced sensitivity to said antibacterial compound to form a mixture; b) incubating said mixture under optimal growth conditions; c) diluting said mixture with an aqueous solution of signal generating reagents, specially selected to detect at least one metabolic marker existing in said bacterium; d) incubating said mixture for sufficient time for said time signal to be detected; d) measuring or detecting said signal in said mixture; e) comparing said signal to a control specimen free of said antibacterial compound, which was simultaneously subjected to steps (a) to (d).

The patent application WO2005/108626, and documents: Eucast et AL, "Determination of minimum inhibitory concentrations (MICs) of antibacterial agents by broth dilution", Clinical Microbiology and Infection, (20030801), pages 1 - 7; and, T.O. OMOTADE ET AL, "D-cycloserine or similar physiochemical compounds may be uniquely suited for use in Bacillus anthracis spore decontamination strategies", JOURNAL OF APPLIED MICROBIOLOGY., GB, (20130830), vol. 115, no. 6, doi:10.1111/jam.12322, ISSN 1364-5072, pages 1343 - 1356; disclose MICs determination and broth dilution methods involving *S. aureus,* in volumes higher than 10-20 μL.

Therefore, it would be advantageous to provide a highly sensitive microbiological method for determining antibiotics, based on low volume biological samples (10-20 μL), without any previous preparation.

**Brief description of the figures**

Figure 1 shows the distribution curves for *S. aureus* growth inhibition (%) depending on the concentration of five antibiotics, assayed three times each. Points are mean ± standard deviation (SD).

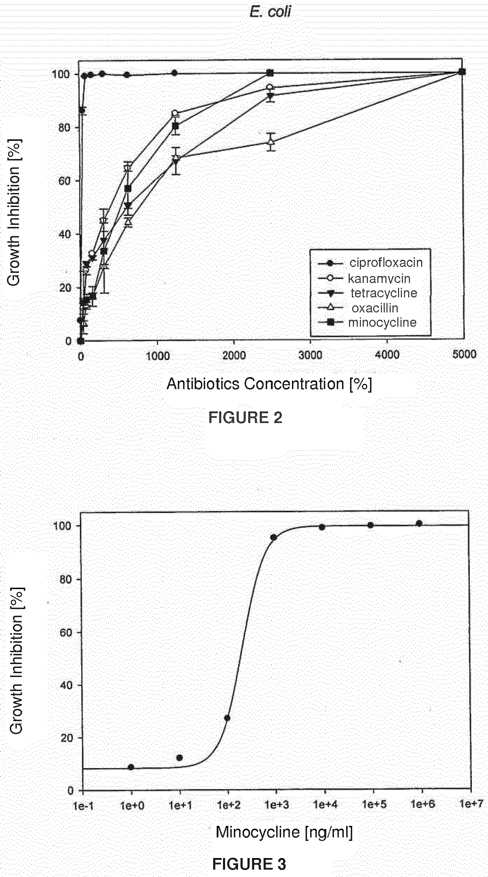

Figure 2 shows the distribution curves of *E. coli* growth inhibition (%) depending on the concentration of five antibiotics tested. It may be seen that *E. coli* was less sensitive to all antibiotics tested. Therefore, the remaining experiments were conducted using S. *aureus* (each antibiotic being assayed three times; the points are mean ± SD).

Figure 3 shows the curve resulting from the application of 4PL curve equation to minocycline antibiotic obtained from Figure 1. From it, Figure 4 curve was drawn.

Figure 4 shows the kinetics of minocycline injected into the cisterna magna. It can be seen that the present method allows quantification of minocycline concentrations in the range of 10-100 ng/ml.

Summary of the invention

The invention is defined in the claims.

**Detailed description of the invention**

**Example**

The minimum inhibitory concentration (MIC) of minocycline, ciprofloxacin, kanamycin, tetracycline and oxacillin antibiotics, was determined by microdilution method in 96-well plates according to the Institute for Clinical Laboratory Standards recommended protocol, *versus* ATCC *Staphylococcus aureus* ATCC 6538 *and Escherichia coli* ATCC 1129. Briefly, bacteria were grown overnight in Mueller-Hinton broth (MHB) and diluted to 0.5 McFarland ($1.5 \times 10^8$ cells/mL). Different antibiotics were added to bacterial cultures in 96-well plates to a final volume of 200 pL. The plates were incubated at 37° C for 18 h and absorbance was determined in an ELISA reader (Thermo Labsystems Multiskan, FC model). The results were expressed as percent inhibition relative to the control containing the cerebrospinal fluid and bacterial culture. Of the two strains of microorganisms tested, Staphylococcus was chosen as the most sensitive to minocycline. The results show that it is possible, for example, to determine minocycline in cerebrospinal fluid (CSF) up to ng/mL level.

A curve representing the percentage of bacterial growth inhibition *versus* antibiotic concentration applied to each well was also obtained. Subsequently, using a curve fit analysis called "Four Parameter Curve Logistic (4PL)", the values of antibiotic concentrations in the biological sample were obtained.

**Example**

**Cerebrospinal fluid (CSF) collection:** It was collected by transcutaneous cisternal magna puncture. Minocycline injections were performed by the same route.

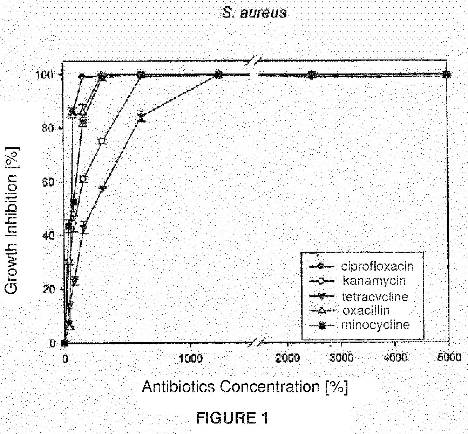

**Curve fitting:** Four Parameter Logistic Curve (4PL) analysis was applied to minocycline antibiotic obtained from Figure 1:

$$y = (max-min)/1 + (x/EC_{50})^{\wedge Hillslope} + min,$$

solving for x:

$$x = EC_{50}((-max+y)/(min-y)^{\wedge(1/Hillslope)}$$

wherein:

min = Bottom of the curve
max = Upper curve
EC$_{50}$ = The value of x for the curve is halfway between the minimum and maximum parameters. It is called the half-maximum effective concentration.
Hillslope = characterizes the slope of the curve at its midpoint.

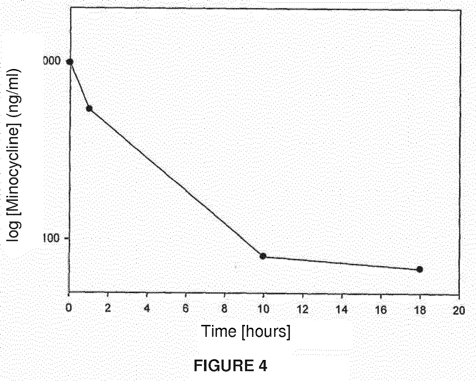

In accordance to the abovementioned, Figure 4 was generated.
Minocycline (MC), an antibiotic belonging to the family of tetracyclines, was used to carry out pharmacokinetic studies of CSF in Sprague-Dawley rats. Briefly, normal rats were injected via intra CSF an MC solution of 1 mg/mL, for subsequent sampling of CSF at time zero, 1, 10 and 18 hours. It may be observed that MC is detected at all times. At 18 hours it is still possible to detect a concentration of 69 ng/mL (Figure 4) .

**Claims**

1. A microbiological method for determining traces of antibiotics in biological samples with a volume of 10-20 microliter, which comprises determining the minimum inhibitory concentration (MIC) of an antibiotic selected from the group consisting of minocycline, ciprofloxacin, kanamycin, tetracycline and oxacillin *versus Staphylococcus aureus,* by growing said bacteria separately in Mueller-Hinton broth (MHB), to subsequently dilute said cultures to 0.5 McFarland standard (1.5 x 10$^8$ cells/mL) and adding afterwards said antibiotics and incubating at 37°C for 18 hours and then determining absorbency and expressing the results as the percentage of inhibition relative to the control containing the biological sample, selected in turn from urine, blood, synovial fluid, eye aqueous humour or cerebrospinal fluid (CSF), and the bacterial culture; obtaining the curve representing the percentage of bacterial growth inhibition *versus* antibiotic concentration, and fitting by the "Four Parameter Logistic Curve (4PL)" curve fitting analysis, to yield the antibiotic concentration values in the biological sample.

2. The method of claim 1, wherein said antibiotic is minocycline.

3. The method of claim 1 wherein the biological sample is cerebrospinal fluid.

4. The method of claim 3 wherein the biological sample of cerebrospinal fluid has been obtained by transcutaneous cisternal magna puncture of a rat.

**Patentansprüche**

1. Mikrobiologisches Verfahren zur Bestimmung von Spuren von Antibiotika in biologischen Proben mit einem Volumen von 10 bis 20 Mikroliter, umfassend das Bestimmen der minimalen Hemmkonzentration (MHK) eines Antibiotikums, ausgewählt aus der Gruppe, bestehend aus Minocyclin, Ciprofloxacin, Kanamycin, Tetracyclin und Oxacillin gegenüber Staphylococcus aureus, durch getrenntes Züchten der Bakterien in Mueller-Hinton-Bouillon (MHB), um nachfolgend die Kulturen auf 0,5 McFarland-Standard (1.5 x 10$^8$ Zellen/ml) zu verdünnen und anschließendes Hinzufügen der Antibiotika und Inkubieren bei 37 °C für 18 Stunden und dann Bestimmen des Absorptionsvermögens und Angeben der Ergebnisse als prozentuale Hemmung relativ zu der Kontrolle, die die biologische Probe enthält, wiederum ausgewählt aus Urin, Blut, Synovialflüssigkeit, Augenkammerwasser oder Zerebrospinalflüssigkeit (CSF), und der Bakterienkultur; Erhalten der Kurve, die den Prozentsatz von Bakterienwachstumshemmung gegenüber Antibiotikakonzentration darstellt, und Anpassen durch die logistische Kurvenanpassungsanalyse mit vier Parametern, um die Antibiotikakonzentrationswerte in der biologischen Probe zu erfassen.

2. Verfahren nach Anspruch 1, wobei das Antibiotikum Minocyclin ist.

3. Verfahren nach Anspruch 1, wobei die biologische Probe Zerebrospinalflüssigkeit ist.

4. Verfahren nach Anspruch 3, wobei die biologische Probe von Liquor cerebrospinalis durch transkutane zisternale Punktion der Magna einer Ratte erhalten wurde.

**Revendications**

1. Procédé microbiologique pour la détermination de traces d'antibiotiques dans les échantillons biologiques ayant un volume de 10-20 microlitres, qui comprend la détermination de la concentration minimale inhibitrice (CMI) d'un antibiotique choisi dans le groupe consistant en minocycline, ciprofloxacine, kanamycine, tétracycline et oxacilline *versus Staphylococcus aureus,* en cultivant ladite bactérie séparément dans Mueller-Hinton broth (MHB), pour diluer ultérieurement lesdites cultures à 0,5 McFarlant standard (1,5 x 10$^8$ cellules/mL) et l'ajout ultérieur desdits antibiotiques et l'incubation à 37 ºC pendant 18 heures puis la détermination de l'absorbance et l'expression des résultats comme le pourcentage d'inhibition relatif au témoin contenant l'échantillon biologique, choisi à son tour parmi l'urine, le sang, le liquide synovial, l'humeur oculaire aqueuse ou le liquide céphalorachidien (LCR) et la culture bactérienne ; l'obtention de la courbe représentant le pourcentage de l'inhibition de la croissance bactérienne *versus* la concentration antibiotique, et l'ajustement par l'analyse d'ajustement de la courbe « courbe logistique à quatre paramètres (4PL) », pour rapporter les valeurs de concentration antibiotique dans l'échantillon biologique.

2. Procédé selon la revendication 1, dans lequel ledit antibiotique est la minocycline.

3. Procédé selon la revendication 1 dans lequel l'échantillon biologique est le liquide céphalorachidien.

4. Procédé selon la revendication 3 dans lequel l'échantillon biologique de liquide céphalorachidien a été obtenu par piqûre transcutanée de la grande citerne d'un rat.

FIGURE 1

FIGURE 2

FIGURE 3

FIGURE 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 1858232
- EP 0418113 A
- WO 2005108626 A

**Non-patent literature cited in the description**

- **S. FAINE ; D.C. KNIGHT.** Rapid microbiological assay of antibiotics in blood and other body fluids. *The Lancet,* 17 August 1968, 375-378
- **MARIE-HÉLÈNE LE BRETON ; MARIE-CLAUDE ; SAVOY-PERROUD ; JEAN-MARC DISERENS.** Validation and comparison of the Copan Milk Test and Delvotest SP-NT for the detection of antimicrobials in milk. *Analytica Chimica Acta,* 2007, vol. 586 (1-2), 280-283
- **ANDRES F. ZULUAGA ; MARIA AGUDELO ; CARLOS RODRIGUEZ ; OMAR VESGA.** *BMC Clinical Pharmacology,* 2009, vol. 9, 1
- **EUCAST et al.** Determination of minimum inhibitory concentrations (MICs) of antibacterial agents by broth dilution. *Clinical Microbiology and Infection,* 1-7
- **T.O. OMOTADE et al.** D-cycloserine or similar physiochemical compounds may be uniquely suited for use in Bacillus anthracis spore decontamination strategies. *JOURNAL OF APPLIED MICROBIOLOGY,* 30 August 2013, vol. 115 (6), ISSN 1364-5072, 1343-1356